# EUROPEAN PATENT SPECIFICATION

(11) **EP 0 811 596 B1**
(45) Date of publication and mention of the grant of the patent: **25.07.2001**
(21) Application number: 97303658.5
(22) Date of filing: 29.05.1997
(51) Int. Cl.: C07C 43/29, C07C 41/30, C07C 25/24

(54) **Process and intermediate compounds for the preparation of pesticidal fluoroolefin compounds**
Verfahren und Zwischenprodukte zur Herstellung von Pestiziden-Fluorolefinverbindungen
Procédé et intermédiaires pour la préparation de composés fluorooléfiniques pesticides

(30) Priority: 03.06.1996 US 657268
(43) Date of publication of application: 10.12.1997
(73) Proprietor: BASF AKTIENGESELLSCHAFT, 67056 Ludwigshafen (DE)
(72) Inventor: Barnes, Keith Douglas, Newtown, Pennsylvania 18940 (US); Hu, Yulin, Plainsboro, New Jersey 08536 (US); Hunt, David Allen, Newtown, Pennsylvania 18940 (US)
(74) Representative: Langfinger, Klaus Dieter, Dr.

(56) References cited:
- GB-A- 2 270 693
- GB-A- 2 288 803
- JOURNAL OF ORGANIC CHEMISTRY, vol. 60, no. 8, 21 April 1995, EASTON US, pages 2396-2397, XP002035635 M. MORENO-MANAS ET AL: "Preparation of 1,3-diarylpropenes by phosphine-free palladium(0)-catalyzed Suzuki-type coupling of allyl bromides with arylboronic acids"

## Description

Fluoroolefin compounds which are useful as pesticidal agents are described in WO 94/06741 and GB 2,288,803-A. Those references also describe processes for the preparation of fluoroolefin compounds. However, those processes are not entirely satisfactory because the fluoroolefin compounds are produced in relatively low yields using long reaction periods. Those references also fail to teach how to make the intermediate compounds of the present invention.

J. Org. Chem. 60, 2396-2397 (1995) describes the preparation of 1,3-diarylpropenes in high yields by the reaction of cinnamyl bromides and arylboronic acids using Pd(dba)ₙ (where n=1.5-2) as catalyst in benzene under reflux in the presence of potassium carbonate. The process has a reaction period of 15 hours and uses 2.15 moles of aryl boronic acid per mole of the cinnamyl bromide.

### SUMMARY OF THE INVENTION

The present invention provides an effective and efficient process for the preparation of a pesticidal fluoroolefin compound having the structural formula I 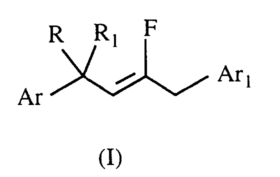 wherein
- R: is hydrogen or C₁-C₄alkyl, and
- R₁: is C₁-C₄alkyl or cyclopropyl, or R and R₁ are taken together with the carbon atom to which they are attached to form a cyclopropyl group;
- Ar: is phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups;
- Ar₁: is phenoxyphenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups, or
benzoylphenyl optionally substituted with any combination of from one to five halogen, C₁-C₄alkyl, C₁-C₄haloalkyl, C₁-C₄alkoxy or C₁-C₄haloalkoxy groups; and
the configuration of the groups ArCRR₁- and -CH₂Ar₁ about
the double bond is predominately mutually trans, which process comprises reacting a 4-aryl-2-fluoro-2-butene-1-ol compound having the structural formula II 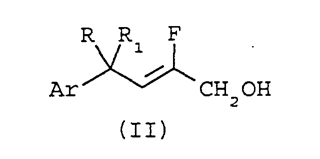 wherein Ar, R and R₁ are as described above, with a brominating agent to form a 4-aryl-1-bromo-2-fluoro-2-butene compound having the structural formula III 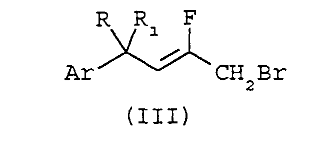 wherein Ar, R and R₁ are as described above, and reacting the formula III compound with a palladium catalyst, a base, and a boronic acid having the structural formula IV, a boronic anhydride having the structural formula V or a borate ester having the structural formula VI

(HO)₂B-Ar₁ (IV)

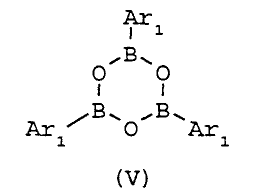

(R₂O)₂B-Ar₁ (VI)

wherein R₂ is C₁-C₄alkyl and Ar₁ is as described above.

The present invention also relates to the intermediate 4-aryl-1-bromo-2-fluoro-2-butene compounds of formula III.

It is, therefore, an object of the present invention to provide an effective and efficient process for the preparation of pesticidal fluoroolefin compounds.

It is also an object of the present invention to provide intermediate compounds which are useful for the preparation of pesticidal fluoroolefin compounds.

Other objects and advantages of the present invention will be apparent to those skilled in the art from the description below and the appended claims.

### DETAILED DESCRIPTION OF THE INVENTION

A preferred embodiment of the present invention comprises reacting a 4-aryl-2-fluoro-2-butene-1-ol of formula II with at least one molar equivalent of a brominating agent, preferably in a temperature range of 50 °C to 130 °C, in the presence of a first solvent to form a 4-aryl-1-bromo-2-fluoro-2-butene of formula III, and reacting the formula III compound with 0.001 to 0.1, preferably 0.005 to 0.1, molar equivalent of a palladium catalyst, at least 2 molar equivalents, preferably 2-6 molar equivalents of a base, and a boronic acid of formula IV, preferably 1 molar equivalent of a boronic acid in a temperature range of 50 °C to 130 °C, in the presence of a second solvent.

The present invention also relates to the 4-aryl-1-bromo-2-fluoro-2-butene compounds which are utilized in the process of this invention. The 4-aryl-1-bromo-2-fluoro-2-butene compounds have the structural formula III 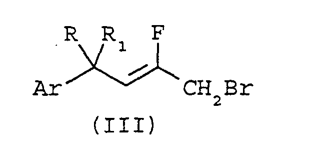 wherein
- R: is hydrogen or C₁-C₄alkyl, and
- R₁: is C₁-C₄alkyl or cyclopropyl, or R and R₁ are taken together with the carbon atom to which they are attached to form a cyclopropyl group;
- Ar: is phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups; and
the configuration of the groups ArCRR₁- and -CH₂Br about
the double bond is predominately mutually trans.

Exemplary of halogen hereinabove are fluorine, chlorine, bromine and iodine. The terms "C₁-C₄haloalkyl" and "C₁-C₄haloalkoxy" are defined as a C₁-C₄alkyl group and a C₁-C₄alkoxy group substituted with one or more halogen atoms, respectively.

The product formula I compounds may be isolated by diluting the reaction mixture with water and extracting the product with a suitable extraction solvent. In the isolation procedure, conventional extraction solvents such as ether, ethyl acetate, toluene and methylene chloride may be utilized.

Brominating agents suitable for use in the process of this invention include, but are not limited to, bromine/triphenyl phosphine complexes, phosphorus tribromide, thionyl bromide and concentrated hydrobromic acid and mixtures thereof. Bromine/triphenyl phosphine complexes are preferred brominating agents.

First solvents suitable for use in the present invention include, but are not limited to, aromatic hydrocarbons such as toluene, benzene, xylenes, mesitylene, halogenated aromatic hydrocarbons such as chlorobenzene, fluorobenzene , carboxylic acid amides such as N,N-dimethylformamide, ethers such as tetrahydrofuran, dioxane, and halogenated hydrocarbons such as chloroform, carbon tetrachloride, and mixtures thereof. Preferred first solvents include halogenated hydrocarbons with carbon tetrachloride being more preferred.

Second solvents useful in the process of this invention include, but are not limited to, aromatic hydrocarbons such as toluene, benzene, xylenes, mesitylene, halogenated aromatic hydrocarbons such as chlorobenzene, fluorobenzene , carboxylic acid amides such as N,N-dimethylformamide, glycols such as dimethoxyethane, C₁-C₄alcohols such as methanol, ethanol, ketones such as acetone, and ethers such as tetrahydrofuran, dioxane, and mixtures thereof, and mixtures with water. Preferred second solvents include aromatic hydrocarbons and aromatic hydrocarbon/C₁-C₄alcohol mixtures with a toluene/ethanol mixture being more preferred.

Palladium catalysts suitable for use in the present invention include, but are not limited to, palladium(0) catalysts such as bis(dibenzylideneacetone)palladium(0), tetrakis(triphenylphosphine)palladium(0), palladium(II) catalysts such as bis(acetonitrile)-palladium(II) chloride, bis(triphenylphosphine)-palladium(II) chloride, [1,4-bis(diphenylphosphine)-butane]palladium(II) dichloride, [1,1'-bis(diphenylphosphino)ferrocene]palladium(II) diacetate, palladium(II) acetate, palladium(II) chloride and the like, and palladium on activated carbon, and mixtures thereof. Preferred catalysts include palladium(0) catalysts with bis(dibenzylideneacetone)palladium(0) being more preferred.

Bases suitable for use in this invention include, but are not limited to, alkali metal carbonates such as sodium carbonate and potassium carbonate, alkaline earth metal carbonates such as calcium carbonate, alkali metal hydrogen carbonates such as sodium hydrogen carbonate, alkali metal hydroxides such as sodium hydroxide and potassium hydroxide, alkaline earth metal hydroxides such as calcium hydroxide, alkali metal C₁-C₆alkoxides such as potassium *tert*-butoxide, thallium(I) carbonate, thallium(I) C₁-C₆alkoxides, thallium(I) hydroxide, and tri(C₁-C₄alkyl)amines such as trimethylamine, and mixtures thereof. Preferred bases include alkali metal carbonates with potassium carbonate being more preferred.

In another preferred embodiment of the present invention, a 4-aryl-1-bromo-2-fluoro-2-butene of formula III is reacted with a palladium catalyst, a base and a boronic acid of formula IV.

Boronic acids of formula IV, boronic anhydrides of formula V, and borate esters of formula VI may be prepared, as illustrated in Flow Diagram I, by reacting an aryl compound of formula VII with a tri(C₁-C₄alkyl) borate of formula VIII to form the formula VI borate ester, and hydrolyzing the formula VI borate ester with aqueous acid to form the boronic acid of formula IV which may spontaneously combine with other formula IV compounds to form the boronic anhydride of formula V.

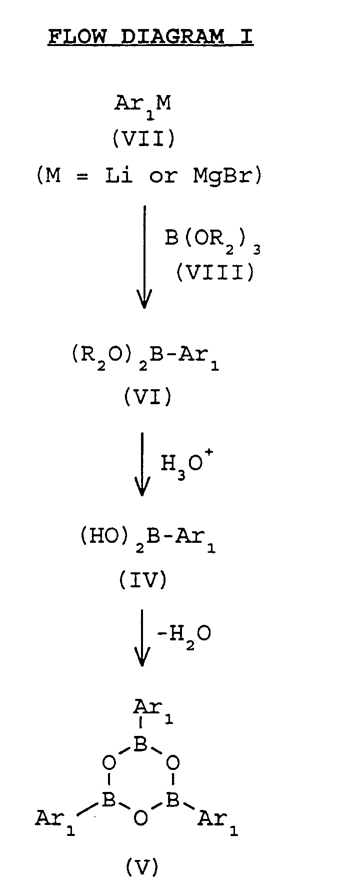

Starting 4-aryl-2-fluoro-2-butene-1-ol compounds of formula II may be prepared according to the procedures described in WO 94/06741 and GB 2,288,803-A.

In another preferred embodiment of the present invention, a 4-aryl-2-fluoro-2-butene-1-ol of formula II is reacted with a brominating agent in a temperature range of 50 °C to 130 °C, and a 4-aryl-1-bromo-2-fluoro-2-butene of formula III is reacted with a palladium catalyst, a base and a formula IV, V or VI compound in a temperature range of 50 °C to 130 °C.

Preferred formula I fluoroolefin compounds which may be prepared by the process of this invention are those wherein
- R: is hydrogen and R₁ is isopropyl or cyclopropyl, or R and R₁ are methyl, or R and R₁ are taken together with the carbon atom to which they are attached to form a cyclopropyl group;
- Ar: is phenyl optionally substituted with any combination of from one to three halogen, C₁-C₄alkyl, C₁-C₄haloalkyl, C₁-C₄alkoxy or C₁-C₄haloalkoxy groups; and
- Ar₁: is 3-phenoxyphenyl optionally substituted with any combination of from one to five halogen, C₁-C₄alkyl, C₁-C₄haloalkyl, C₁-C₄alkoxy or C₁-C₄haloalkoxy groups,
3-biphenyl optionally substituted with any combination of from one to five halogen, C₁-C₄alkyl, C₁-C₄haloalkyl, C₁-C₄alkoxy or C₁-C₄haloalkoxy groups,
3-benzylphenyl optionally substituted with any combination of from one to five halogen, C₁-C₄alkyl, C₁-C₄haloalkyl, C₁-C₄alkoxy or C₁-C₄haloalkoxy groups, or
3-benzoylphenyl optionally substituted with any combination of from one to five halogen, C₁-C₄alkyl, C₁-C₄haloalkyl, C₁-C₄alkoxy or C₁-C₄haloalkoxy groups.

The process of the present invention is also preferably used for the preparation of pesticidal fluoroolefins of formula I wherein
- R and R₁: are methyl, or R and R₁ are taken together with the carbon atom to which they are attached to form a cyclopropyl group;
- Ar: is phenyl optionally substituted with any combination of from one to three halogen, C₁-C₄alkyl, C₁-C₄haloalkyl, C₁-C₄alkoxy or C₁-C₄haloalkoxy groups; and
- Ar₁: is 3-phenoxyphenyl optionally substituted with any combination of from one to five halogen, C₁-C₄alkyl, C₁-C₄haloalkyl, C₁-C₄alkoxy or C₁-C₄haloalkoxy groups.

In order to facilitate a further understanding of the invention, the following examples are presented primarily for the purpose of illustrating more specific details thereof. The scope of the invention should not be deemed limited by the examples, but encompasses all the subject matter defined in the claims.

### EXAMPLE 1

### Preparation of 1-(p-Chlorophenyl)-1-(3-bromo-2-fluoropropenyl)cyclopropane, (Z)

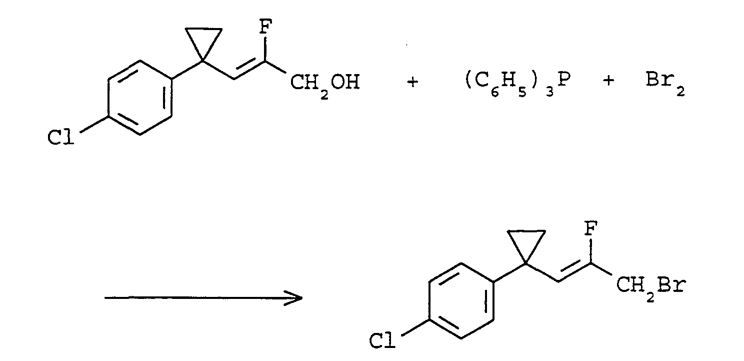

Under a nitrogen atmosphere, a solution of bromine (0.767 g, 4.8 mmol) in carbon tetrachloride at ice-water bath temperature is treated sequentially with a solution of triphenylphosphine (1.26 g, 4.8 mmol) in carbon tetrachloride and a solution of 3-[1-(*p*-chlorophenyl)-cyclopropyl]-2-fluoro-2-propen-1-ol, (Z)- (0.907 g, 4 mmol) in carbon tetrachloride, heated to and stirred at reflux for 70 minutes, cooled to room temperature, and poured into petroleum ether. The resultant mixture is filtered, and the filtrate is concentrated *in vacuo* to obtain a residue. The residue is dissolved in petroleum ether and passed through a plug of silica gel (eluted with hexanes) to obtain an oil. Flash column chromatography of the oil using silica gel and a 3:20 methylene chloride/hexanes solution gives the title product as a colorless oil (0.73 g, 63% yield) which is identified by NMR spectral analyses.

### EXAMPLE 2

### Preparation of 1-(p-Chlorophenyl)-1-[2-fluoro-3-(4-fluoro-3-phenoxyphenyl)propenyl]cyclopropane, (Z)-

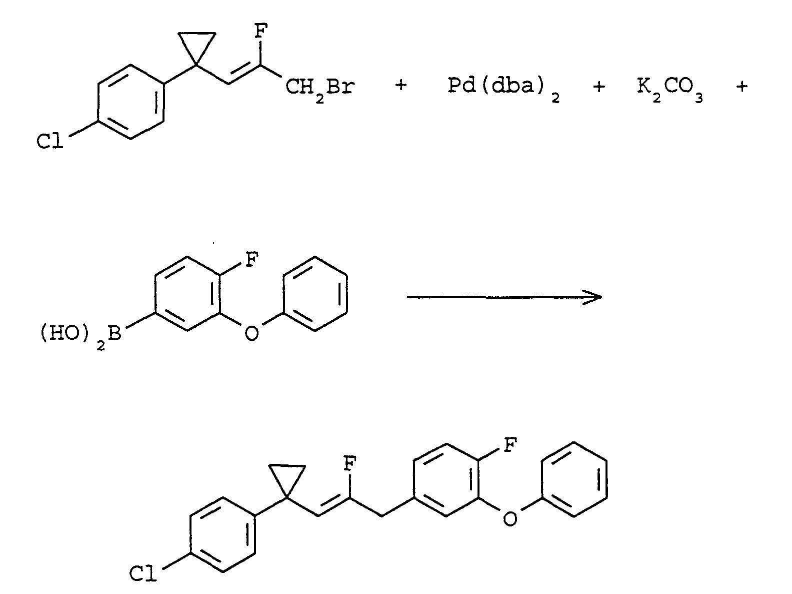

Under a nitrogen atmosphere, a mixture of 1-(*p*-chlorophenyl)-1-(3-bromo-2-fluoropropenyl)cyclopropane, (Z)- (0.434 g, 1.5 mmol), 4-fluoro-3-phenoxybenzeneboronic acid (0.452 g, 1.95 mmol), potassium carbonate (1.86 g, 13.5 mmol) and bis(dibenzylideneacetone)-palladium(0) (Pd(dba)₂, 4.3 mg, 0.075 mmol) in toluene is heated at 80 °C for one hour, cooled to room temperature, diluted with water, and filtered through diatomaceous earth. The phases are separated and the aqueous phase is extracted with ethyl acetate. The organic phase and-ethyl acetate extract are combined, washed with water, dried over anhydrous sodium sulfate and concentrated *in vacuo* to obtain a residue. Flash column chromatography of the residue using silica gel and a 3:20 methylene chloride/hexanes solution gives the title product as an oil (0.274 g, 63% yield) which is identified by NMR spectral analyses.

As can be seen from the data in Examples 1 and 2, 1-(*p*-chlorophenyl)-1-[2-fluoro-3-(4-fluoro-3-phenoxyphenyl)propenyl]cyclopropane, (Z)- is obtained in 40% yield from 3-[1-(*p*-chlorophenyl)cyclopropyl]-2-fluoro-2-propen-1-ol, (Z)-. In contrast, WO 94/06741 discloses that 1-(*p*-chlorophenyl)-1-[2-fluoro-3-(4-fluoro-3-phenoxyphenyl)propenyl]cyclopropane, (Z)- is obtained in 20% yield from 3-[1-(*p*-chlorophenyl)cyclopropyl]-2-fluoro-2-propen-1-ol, (Z)-.

### EXAMPLE 3

### Preparation of 1-Bromo-4-(p-chlorophenyl)-2-fluoro-4-methyl-2-pentene, (Z)-

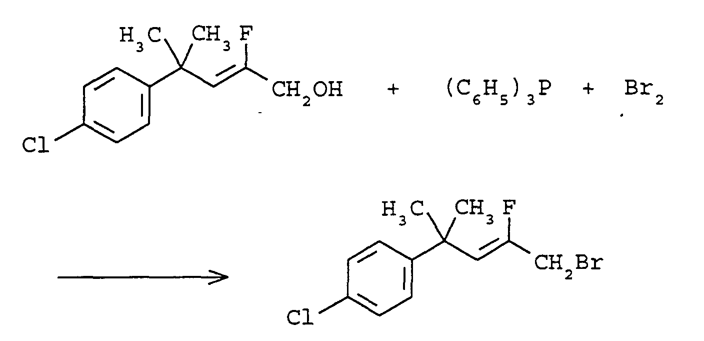

Under a nitrogen atmosphere, a solution of triphenyl phosphine (0.96 g, 4.25 mmol) in carbon tetrachloride at -5 °C is treated dropwise with a solution of bromine (0.679 g, 4.25 mmol) in carbon tetrachloride, warmed to and stirred at room temperature for 45 minutes, treated with a solution of 4-(*p*-chlorophenyl)-2-fluoro-4-methyl-2-penten-1-ol, (Z)- (0.81 g, 3.54 mmol) in carbon tetrachloride, refluxed for 2 hours, cooled to room temperature, and poured into petroleum ether. The resultant mixture is filtered through diatomaceous earth. The filtrate is washed sequentially with water, saturated sodium hydrogen carbonate solution and brine, dried over anhydrous sodium sulfate and concentrated *in vacuo* to obtain an oil. Flash column chromatography of the oil using silica gel and a 1:9 ethyl acetate/hexanes solution gives the title product as a colorless oil (0.736 g, 71% yield) which is identified by NMR spectral analyses.

### EXAMPLE 4

### Preparation of 4- (p-Chlorophenyl)-2-fluoro-1-(4-fluoro-3-phenoxyphenyl)-4-methyl-2-pentene, (Z)-

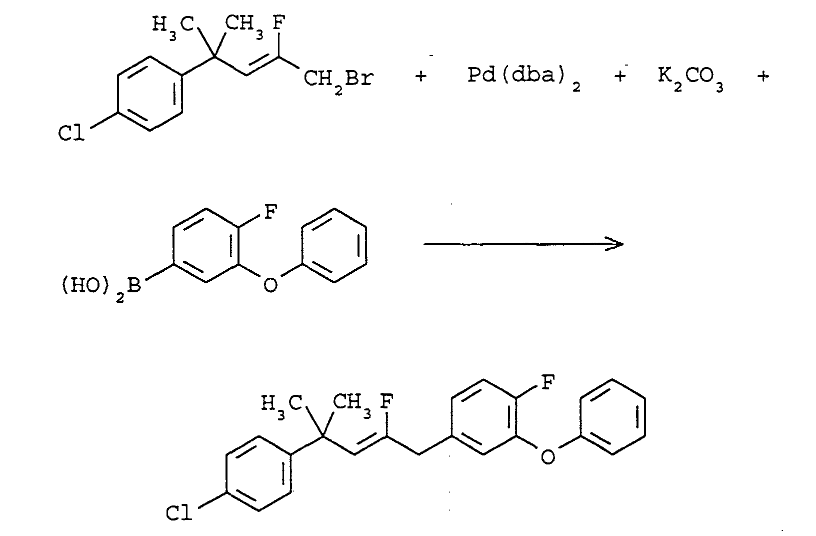

Under a nitrogen atmosphere, a mixture of 1-bromo-4-(*p*-chlorophenyl)-2-fluoro-4-methyl-2-pentene, (Z)- (320.8 mg, 1.1 mmol), bis(dibenzylideneacetone)palladium(0) (Pd(dba)₂, 31.6 mg, 0.055 mmol) in toluene (8 mL) is treated with potassium carbonate (608 mg, 4.4 mmol), degassed, treated with a solution of 4-fluoro-3-phenoxybenzeneboronic acid (331.8 mg, 1.43 mmol) in ethanol (2 mL), refluxed for 45 minutes, cooled to room temperature, and filtered through diatomaceous earth. The filtrate is diluted with ethyl acetate, and the resultant solution is washed sequentially with water and brine, dried over anhydrous sodium sulfate, and concentrated *in vacuo* to obtain a residue. Flash column chromatography of the residue using silica gel and a 1:9 ethyl acetate/hexanes solution gives the title product as a colorless liquid (401 mg, 91% yield) which is identified by NMR spectral analyses.

As can be seen from the data in Examples 3 and 4, 4-(*p*-chlorophenyl)-2-fluoro-1-(4-fluoro-3-phenoxyphenyl)-4-methyl-2-pentene, (Z)- is obtained in 65% yield from 4-(*p*-chlorophenyl)-2-fluoro-4-methyl-2-penten-1-ol, (Z)-. In contrast, GB 2,288,803-A discloses that 4-(*p*-chlorophenyl)-2-fluoro-1-(4-fluoro-3-phenoxyphenyl)-4-methyl-2-pentene, (Z)- is obtained in 37% yield from 4-(*p*-chlorophenyl)-2-fluoro-4-methyl-2-penten-1-ol, (Z)-.

### EXAMPLE 5

### Preparation of 4-Fluoro-3-phenoxybenzeneboronic acid

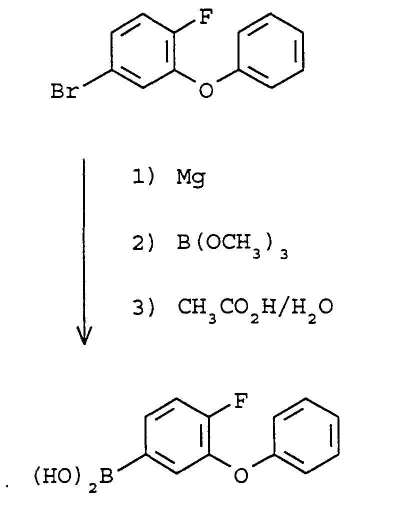

A solution of 5-bromo-2-fluorophenyl phenyl ether (8.01 g, 3 mmol) in tetrahydrofuran is added dropwise to a mixture of magnesium turnings (0.0802 g, 3.3 mmol), a crystal of iodine and a few drops of 1,2-dibromoethane in tetrahydrofuran at 50-55 °C under nitrogen. After the addition is complete, the reaction mixture is stirred at 50-55 °C for 70 minutes and cooled to room temperature. The cooled mixture is added over 25 minutes to a solution of trimethyl borate (4.09 mL, 3.6 mmol) in diethyl ether at dry-ice/acetone bath temperature. After the addition is complete, the mixture is stirred at dry-ice/acetone bath temperature for 20 minutes, allowed to warm to -10 °C over 25 minutes, diluted sequentially with acetic acid and water, stirred at room temperature for 30 minutes, and extracted with ether. The organic extract is washed with water, dried over anhydrous sodium sulfate, and concentrated *in* vacuo to obtain a residue. A mixture of the residue in water is heated over a steam bath for 30 minutes, cooled to room temperature and filtered to obtain a solid which is washed with hexanes and dried to give the title product as a colorless solid (5.7 g, mp 177-180 °C, 82% yield).

## Claims

1. A process for the preparation of a fluoroolefin compound having the structural formula I 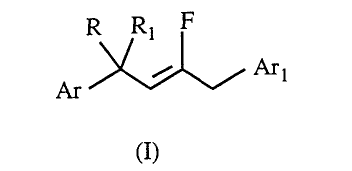 wherein
R is hydrogen or C₁-C₄alkyl, and
R₁ is C₁-C₄alkyl or cyclopropyl, or R and R₁ are taken together with the carbon atom to which they are attached to form a cyclopropyl group;
Ar is phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups;
Ar₁ is phenoxyphenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups, or
benzoylphenyl optionally substituted with any combination of from one to five halogen, C₁-C₄alkyl, C₁-C₄haloalkyl, C₁-C₄alkoxy or C₁-C₄haloalkoxy groups; and
the configuration of the groups ArCRR₁- and -CH₂Ar₁ about
the double bond is predominately mutually trans, which process comprises reacting a 4-aryl-2-fluoro-2-butene-1-ol compound having the structural formula II 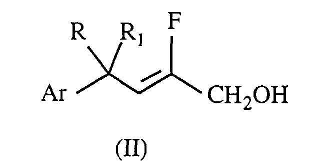 wherein Ar, R and R₁ are as described above with a brominating agent to form a 4-aryl-1-bromo-2-fluoro-2-butene compound having the structural formula III 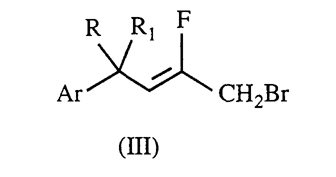 wherein Ar, R and R₁ are as described above, and reacting the formula III compound with a palladium catalyst, a base, and a boronic acid having the structural formula IV, a boronic anhydride having the structural formula V or a borate ester having the structural formula VI
(HO)₂B-Ar₁ (IV) 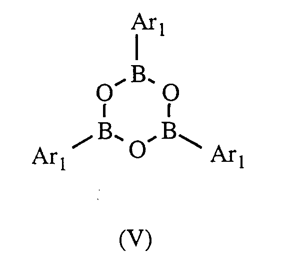
(R₂O)₂B-Ar₁ (VI)
wherein R₂ is C₁-C₄alkyl and Ar₁ is as described above.

2. The process according to claim 1 wherein the brominating agent is a bromine/triphenylphosphine complex.

3. The process according to claim 1 or 2 wherein the palladium catalyst is selected from the group consisting of bis(dibenzylideneacetone)palladium(0), tetrakis(triphenylphosphine)palladium(0), bis(acetonitrile)palladium(II) chloride, bis(triphenylphosphine)palladium(II) chloride, [1,4-bis(diphenylphosphine)butane]palladium(II) dichloride, [1,1'-bis(diphenylphosphino)ferrocene]palladium(II) diacetate, palladium(II) acetate, palladium(II) chloride and palladium on activated carbon and mixtures thereof; and the base is selected from the group consisting of an alkali metal carbonate, an alkaline earth metal carbonate, an alkali metal hydrogen carbonate, an alkali metal hydroxide, an alkaline earth metal hydroxide, an alkali metal C₁-C₆alkoxide, thallium(I) carbonate, a thallium(I) C₁-C₆alkoxide, thallium(I) hydroxide and a tri(C₁-C₄alkyl)amine and mixtures thereof.

4. The process according to claim 3 wherein the palladium catalyst is bis(dibenzylideneacetone)palladium(0) and the base is an alkali metal carbonate.

5. The process according to claim 1 wherein the formula III compound is reacted with a boronic acid.

6. The process according to any preceding claim wherein the first reaction step is carried out in a first solvent selected from the group consisting of an aromatic hydrocarbon, a halogenated aromatic hydrocarbon, a carboxylic acid amide, an ether and a halogenated hydrocarbon and mixtures thereof; and the last reaction step is carried out in a second solvent selected from the group consisting of an aromatic hydrocarbon, a halogenated aromatic hydrocarbon, a carboxylic acid amide, a glycol, a C₁-C₄alcohol, a ketone and an ether and mixtures thereof and mixtures with water.

7. The process according to claim 6 wherein the first solvent is a halogenated hydrocarbon and the second solvent is toluene or a toluene/ethanol mixture.

8. The process according to any preceding claim wherein the formula II compound is reacted with the brominating agent at a temperature of 50°C to 130°C, and the formula III compound is reacted with the palladium catalyst, the base and the formula IV, V or VI compound at a temperature of 50°C to 130°C.

9. A process for the preparation of a fluoroolefin compound having the structural formula I 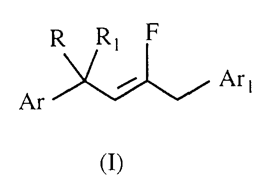 wherein
R is hydrogen or C₁-C₄alkyl, and
R₁ is C₁-C₄alkyl or cyclopropyl, or R and R₁ are taken together with the carbon atom to which they are attached to form a cyclopropyl group;
Ar is phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups;
Ar₁ is phenoxyphenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups, or
benzoylphenyl optionally substituted with any combination of from one to five halogen, C₁-C₄alkyl, C₁-C₄haloalkyl, C₁-C₄alkoxy or C₁-C₄haloalkoxy groups; and
the configuration of the groups ArCRR₁- and -CH₂Ar₁ about the
double bond is predominately mutually trans, which process comprises reacting a 4-aryl-1-bromo-2-fluoro-2-butene compound having the structural formula III 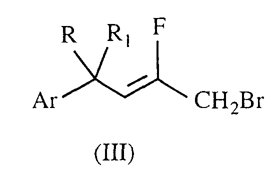 wherein Ar, R and R₁ are as described above with 0.001 to 0.1 molar equivalent of a palladium catalyst, at least 2 molar equivalents of a base, and a boronic acid having the structural formula IV, a boronic anhydride having the structural formula V or a borate ester having the structural formula VI
(HO)₂B-Ar₁ (IV) 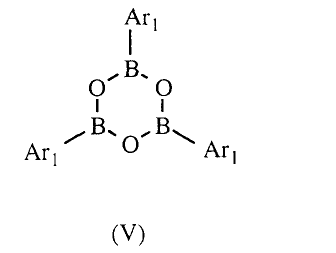
(R₂O)₂B-Ar₁ (VI)
wherein R₂ is C₁-C₄alkyl and Ar₁ is as described above, at a temperature of 50-130°C in the presence of a solvent.

10. The process according to claim 9 wherein the palladium catalyst is selected from the group consisting of bis(dibenzylideneacetone)palladium(0), tetrakis(triphenylphosphine)palladium(0), bis(acetonitrile)palladium(II) chloride, bis(triphenylphosphine)palladium(II) chloride, [1,4-bis(diphenylphosphine)butane]palladium(II) dichloride, [1,1'-bis(diphenylphosphino)ferrocene]palladium(II) diacetate, palladium(II) acetate, palladium(II) chloride and palladium on activated carbon and mixtures thereof; and the base is selected from the group consisting of an alkali metal carbonate, an alkaline earth metal carbonate, an alkali metal hydrogen carbonate, an alkali metal hydroxide, an alkaline earth metal hydroxide, an alkali metal C₁-C₆alkoxide, thallium(I) carbonate, a thallium(I) C₁-C₆alkoxide, thallium(I) hydroxide and a tri(C₁-C₄alkyl)-amine and mixtures thereof.

11. The process according to claim 10 wherein the palladium catalyst is bis(dibenzylideneacetone)palladium(0) and the base is an alkali metal carbonate.

12. The process according to any one of claims 9 to 11 wherein the solvent is selected from the group consisting of an aromatic hydrocarbon, a halogenated aromatic hydrocarbon, a carboxylic acid amide, a glycol, a C₁-C₄alcohol, a ketone and an ether and mixtures thereof and mixtures with water.

13. A compound having the structural formula III 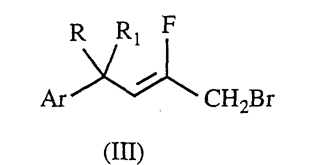 wherein
R is hydrogen or C₁-C₄alkyl, or R and R₁ are taken together with the carbon atom to which they are attached to form a cyclopropyl group;
R₁ is C₁-C₄alkyl or cyclopropyl, or R and R₁are taken together with the carbon atom to which they are attached to form a cyclopropyl group;
Ar is phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups; and
the configuration of the groups ArCRR₁ and -CH₂Br about the double bond being predominately mutually trans.

14. The compound according to claim 13 selected from the group consisting of
1-(*p*-chlorophenyl)-1-(3-bromo-2-fluoropropenyl)cyclopropane, (Z)-; and
1-bromo-4-(*p*-chlorophenyl)-2-fluoro-4-methyl-2-pentene, (Z)-.

15. A process for making a compound according to claim 13 which process comprises reacting a 4-aryl-2-fluoro-2-butene-1-ol compound having structural formula II 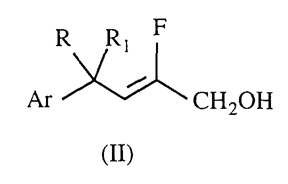 wherein R, R₁ and Ar are as defined in claim 17, with a brominating agent selected from the group consisting of a bromine-triphenyl phosphine complex, phosphorus tribromide, thionyl bromide, HBr, and mixtures thereof, in a solvent at a temperature of 50-130°C.

## Patentansprüche

1. Verfahren zur Herstellung einer Fluorolefinverbindung mit der Strukturformel I 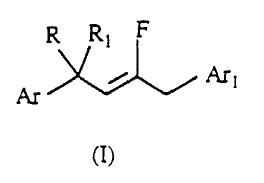 wobei
R für Wasserstoff oder C₁-C₄-Alkyl steht, und
R₁ für C₁-C₄-Alkyl oder Cyclopropyl steht, oder R und R₁ zusammen mit dem Kohlenstoffatom, an das sie gebunden sind, eine Cyclopropylgruppe bilden;
Ar für Phenyl steht, welches gegebenenfalls durch eine beliebige Kombination von einem bis drei Substituenten aus der Gruppe Halogen, C₁-C₄-Alkyl, C₁-C₄-Halogenalkyl, C₁-C₄-Alkoxy oder C₁-C₄-Halogenalkoxy substituiert ist, oder
für1- oder 2-Naphthyl steht, welches gegebenenfalls durch eine beliebige Kombination von einem bis drei Substituenten aus der Gruppe Halogen, C₁-C₄-Alkyl, C₁-C₄-Halogenalkyl, C₁-C₄-Alkoxy oder C₁-C₄-Halogenalkoxy substituiert ist;
Ar₁ für Phenoxyphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist,
für Biphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist,
für Benzylphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Haloaenalkoxy substituiert ist, oder
für Benzoylphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist, und
die Gruppen ArCRR₁- und -CH₂Ar₁ an der Doppelbindung
vorwiegend trans zueinander konfiguriert sind, dadurch gekennzeichnet, daß man bei dem Verfahren eine 4-Aryl-2-fluorbut-2-en-1-ol-Verbindung mit der Strukturformel II 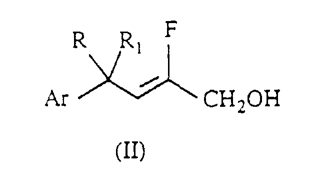 wobei Ar, R und R₁ wie oben beschrieben sind, mit einem Bromierungsmittel zu einer 4-Aryl-1-brom-2-fluorbut-2-en-Verbindung mit der Strukturformel III 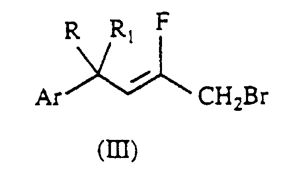 wobei Ar, R und R₁ wie oben beschrieben sind, umsetzt und die Verbindung der Formel III mit einem Palladiumkatalysator, einer Base und einer Boronsäure der Strukturformel IV, einem Boronsäureanhydrid der Strukturformel V oder einem Boronsäureester der Strukturformel VI
(HO)₂B-Ar₁ (IV) 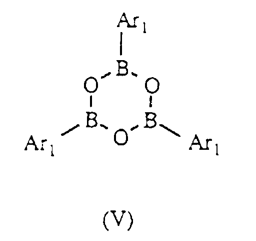
(R₂O)₂B-Ar₁ (VI)
wobei R₂ für C₁-C₄-Alkyl steht und Ar₁ wie oben beschrieben ist, umsetzt.

2. Verfahren nach Anspruch 1, wobei es sich bei dem Bromierungsmittel um einen Brom/Triphenylphosphinkomplex handelt.

3. Verfahren nach Anspruch 1 oder 2, wobei der Palladiumkatalysator aus der Gruppe Bis(dibenzylidenaceton)palladium(0), Tetrakis(triphenylphosphin)palladium(0), Bis(acetonitril)palladium(II)-chlorid, Bis(triphenylphosphin)palladium(II)-chlorid, [1,4-Bis(diphenylphosphin)butan]palladium-(II)-dichlorid, [1,1'-Bis(diphenylphosphino)ferrocen]palladium(II)-diacetat, Palladium(II)-acetat, Palladium(II)-chlorid und Palladium-auf-Aktivkohle sowie Mischungen davon ausgewählt ist; und die Base aus der Gruppe Alkalicarbonat, Erdalkalicarbonat, Alkalihydrogencarbonat, Alkalihydroxid, Erdalkalihydroxid, Alkali-C₁-C₆-alkoholat, Thallium(I)-carbonat, Thallium(I) -C₁-C₆-alkoholat, Thallium(I)-hydroxid und Tri-(C₁-C₄-alkyl)-amin und Mischungen davon ausgewählt ist.

4. Verfahren nach Anspruch 3, wobei es sich bei dem Palladiumkatalysator um Bis(dibenzylidenaceton)-palladium(0) und bei der Base um ein Alkalicarbonat handelt.

5. Verfahren nach Anspruch 1, wobei die Verbindung der Formel III mit einer Boronsäure umgesetzt wird.

6. Verfahren nach einem der vorhergehenden Ansprüche, wobei der erste Reaktionsschritt in einem ersten Lösungsmittel, ausgewählt aus der Gruppe aromatischer Kohlenwasserstoff, halogenierter aromatischer Kohlenwasserstoff, Carbonsäureamid, Ether und Halogenkohlenwasserstoff sowie Mischungen davon, durchgeführt wird; und der letzte Reaktionsschritt in einem zweiten Lösungsmittel, ausgewählt aus der Gruppe aromatischer Kohlenwasserstoff, halogenierter aromatischer Kohlenwasserstoff, Carbonsäureamid, Glykol, C₁-C₄-Alkohol, Keton und Ether sowie Mischungen davon und Mischungen davon mit Wasser, durchgeführt wird.

7. Verfahren nach Anspruch 6, wobei es sich bei dem ersten Lösungsmittel um einen Halogenkohlenwasserstoff und bei dem zweiten Lösungsmittel um Toluol oder eine Toluol/Ethanol-Mischung handelt.

8. Verfahren nach einem der vorhergehenden Ansprüche, wobei die Verbindung der Formel II bei einer Temperatur von 50°C bis 130°C mit dem Bromierungsmittel umgesetzt wird und die Verbindung der Formel III bei einer Temperatur von 50°C bis 130°C mit dem Palladiumkatalysator, der Base und der Verbindung der Formel IV, V oder VI umgesetzt wird.

9. Verfahren zur Herstellung einer Fluorolefinverbindung der Strukturformel I 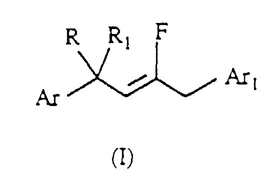 wobei
R für Wasserstoff oder C₁-C₄-Alkyl steht, und
R₁ für C₁-C₄-Alkyl oder Cyclopropyl steht, oder R und R₁ zusammen mit dem Kohlenstoffatom, an das sie gebunden sind, eine Cyclopropylgruppe bilden;
Ar für Phenyl steht, welches gegebenenfalls durch eine beliebige Kombination von einem bis drei Substituenten aus der Gruppe Halogen, C₁-C₄-Alkyl, C₁-C₄-Halogenalkyl, C₁-C₄-Alkoxy oder C₁-C₄-Halogenalkoxy substituiert ist, oder
für 1- oder 2-Naphthyl steht, welches gegebenenfalls durch eine beliebige Kombination von einem bis drei Substituenten aus der Gruppe Halogen, C₁-C₄-Alkyl, C₁-C₄-Halogenalkyl, C₁-C₄-Alkoxy oder C₁-C₄-Halogenalkoxy substituiert ist;
Ar₁ für Phenoxyphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist,
für Biphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist,
für Benzylphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist, oder
für Benzoylphenyl steht, welches gegebenenfalls durch eine beliebige Kombination von einem bis fünf Substituenten aus der Gruppe Halogen, C₁-C₄-Alkyl, C₁-C₄-Halogenalkyl, C₁-C₄-Alkoxy oder C₁-C₄-Halogenalkoxy substituiert ist, und
die Gruppen ArCRR₁- und -CH₂Ar₁ an der Doppelbindung
vorwiegend trans zueinander konfiguriert sind, dadurch gekennzeichnet, daß man eine 4-Aryl-1-brom-2-fluorbut-2-en-Verbindung mit der Strukturformel III 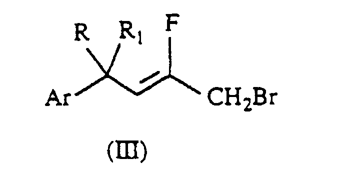 wobei Ar, R und R₁ wie oben beschrieben sind, mit 0,001 bis 0,1 Moläquivalenten eines Palladiumkatalysators, wenigstens 2 Moläquivalenten einer Base und einer Boronsäure der Strukturformel IV, einem Boronsäureanhydrid der Strukturformel V oder einem Boronsäureester der Strukturformel VI
(HO)₂B-Ar₁ (IV) 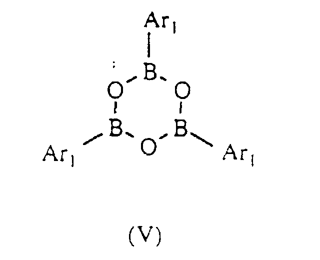
(R₂O)₂B-Ar₁ (VI)
wobei R₂ für C₁-C₄-Alkyl steht und Ar₁ wie oben beschrieben ist, bei einer Temperatur von 50-130°C in Gegenwart eines Lösungsmittels umsetzt.

10. Verfahren nach Anspruch 9, wobei der Palladiumkatalysator aus der Gruppe Bis(dibenzylidenaceton)-palladium(0), Tetrakis(triphenylphosphin)-palladium(0), Bis(acetonitril)palladium(II)-chlorid, Bis(triphenylphosphin)palladium(II)-chlorid, [1,4-Bis(diphenylphosphin)butan]palladium-(II)-dichlorid, [1,1'-Bis(diphenylphosphino)-ferrocen]palladium(II)-diacetat, Palladium(II)-acetat, Palladium(II)-chlorid und Palladium-auf-Aktivkohle sowie Mischungen davon ausgewählt ist; und die Base aus der Gruppe Alkalicarbonat, Erdalkalicarbonat, Alkalihydrogencarbonat, Alkalihydroxid, Erdalkalihydroxid, Alkali-C₁-C₆-alkoholat, Thallium(I) -carbonat, Thallium(I) -C₁-C₆-alkoholat, Thallium(I)-hydroxid und Tri-(C₁-C₄-Alkyl)-amin sowie Mischungen davon ausgewählt ist.

11. Verfahren nach Anspruch 10, wobei es sich bei dem Palladiumkatalysator um Bis(dibenzylidenaceton)-palladium(0) und bei der Base um einen Alkalicarbonat handelt.

12. Verfahren nach einem der Ansprüche 9 bis 11, wobei das Lösungsmittel aus der Gruppe aromatischer Kohlenwasserstoff, halogenierter aromatischer Kohlenwasserstoff, Carbonsäureamid, Glykol, C₁-C₄-Alkohol, Keton und Ether sowie Mischungen davon und Mischungen davon mit Wasser ausgewählt ist.

13. Verbindung der Strukturformel III 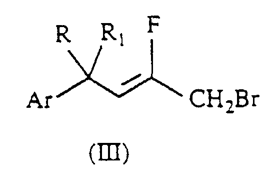 wobei
R für Wasserstoff oder C₁-C₄-Alkyl steht, oder R und R₁ zusammen mit dem Kohlenstoffatom, an das sie gebunden sind, eine Cyclopropylgruppe bilden;
R₁ für C₁-C₄-Alkyl oder Cyclopropyl steht, oder R und R₁ zusammen mit dem Kohlenstoffatom, an das sie gebunden sind, eine Cyclopropylgruppe bilden;
Ar für Phenyl steht, welches gegebenenfalls durch eine beliebige Kombination von einem bis drei Substituenten aus der Gruppe Halogen, C₁-C₄-Alkyl, C₁-C₄-Halogenalkyl, C₁-C₄-Alkoxy oder C₁-C₄-Halogenalkoxy substituiert ist, oder
für 1- oder 2-Naphthyl steht, welches gegebenenfalls durch eine beliebige Kombination von einem bis drei Substituenten aus der Gruppe Halogen, C₁-C₄-Alkyl, C₁-C₄-Halogenalkyl, C₁-C₄-Alkoxy oder C₁-C₄-Halogenalkoxy substituiert ist; und
die Gruppen ArCRR₁- und -CH₂Br an der Doppelbindung vorwiegend trans zueinander konfiguriert sind.

14. Verbindung nach Anspruch 13, ausgewählt aus der Gruppe
1-(*p*-Chlorphenyl)-1-(3-brom-2-fluorpropenyl)-cyclopropan, (Z)-; und
1-Brom-4-(*p*-chlorphenyl)-2-fluor-4-methyl-pent-2-en, (Z)-.

15. Verfahren zur Herstellung einer Verbindung nach Anspruch 13, dadurch gekennzeichnet, daß man eine 4-Aryl-2-fluorbut-2-en-1-ol-Verbindung der Strukturformel II 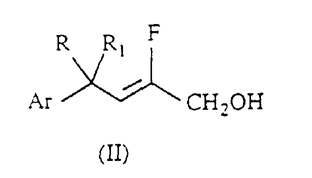 wobei R, R₁ und Ar wie in Anspruch 17 definiert sind, mit einem Bromierungsmittel aus der Gruppe Brom/Triphenylphosphin-Komplex, Phosphortribromid, Thionylbromid, HBr sowie Mischungen davon, in einem Lösungsmittel bei einer Temperatur von 50-130°C umsetzt.

## Revendications

1. Procédé pour la préparation d'un composé oléfinique fluoré ayant la formule structurale I 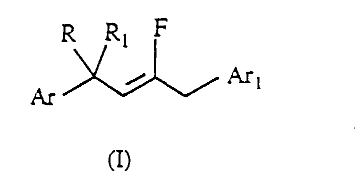 dans laquelle
R est l'hydrogène ou un groupe alkyle en C₁-C₄, et
R₁ est un groupe alkyle en C₁-C₄ ou cyclopropyle, ou bien R et R₁ sont pris avec l'atome de carbone auquel ils sont liés pour former un groupe cyclopropyle ;
Ar est un groupe phényle facultativement substitué par toute association d'un à trois groupes halogéno, alkyle en C₁-C₄, halogénoalkyle en C₁-C₄, alcoxy en C₁-C₄ ou halogénoalcoxy en C₁-C₄, ou
un groupe 1- ou 2-naphtyle facultativement substitué par toute association d'un à trois groupes halogéno, alkyle en C₁-C₄, halogénoalkyle en C₁-C₄, alcoxy en C₁-C₄ ou halogénoalcoxy en C₁-C₄ ;
Ar₁ est un groupe phénoxyphényle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄,
un groupe biphénylyle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄,
un groupe benzylphényle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄, ou
un groupe benzoylphényle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄ ; et
la configuration des groupes ArCRR₁- et -CH₂Ar₁ entre eux
autour de la double liaison est majoritairement *trans*, lequel procédé consiste à faire réagir un composé de 4-aryl-2-fluoro-2-butène-1-ol ayant la formule structurale II 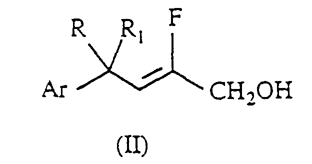 dans laquelle Ar, R et R₁ sont tels que définis ci-dessus, avec un agent de bromation pour former un composé de 4-aryl-1-bromo-2-fluoro-2-butène ayant la formule structurale III 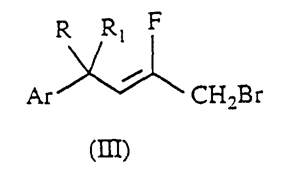 dans laquelle Ar, R et R₁ sont tels que définis ci-dessus, et à faire réagir le composé de formule III avec un catalyseur au palladium, une base et un acide boronique ayant la formule structurale IV, un anhydride boronique ayant la formule structurale V ou un ester borique ayant la formule structurale VI
(HO)₂B-Ar₁ (IV) 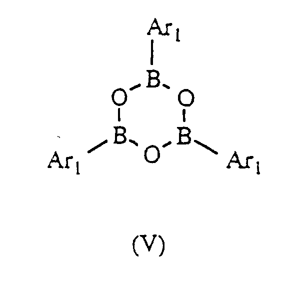
(R₂O)₂B-Ar₁ (VI)
où R₂ est un groupe alkyle en C₁-C₄ et Ar₁ est tel que défini ci-dessus.

2. Procédé selon la revendication 1, dans lequel l'agent de bromation est un complexe brome/triphénylphosphine.

3. Procédé selon la revendication 1 ou 2, dans lequel le catalyseur au palladium est choisi dans la classe formée par le bis(dibenzylidèneacétone)palladium(0), le tétrakis(triphénylphosphine)palladium(0), le chlorure de bis(acétonitrile)palladium(II), le chlorure de bis(triphénylphosphine)palladium(II), le dichlorure de [1,4-bis(diphénylphosphine)butane]palladium(II), le diacétate de [1,1'-bis(diphénylphosphino)ferrocène]palladium(II), l'acétate de palladium(II), le chlorure de palladium(II) et le palladium sur charbon activé, et leurs mélanges ; et la base est choisie dans la classe formée par un carbonate de métal alcalin, un carbonate de métal alcalino-terreux, un hydrogénocarbonate de métal alcalin, un hydroxyde de métal alcalin, un hydroxyde de métal alcalino-terreux, un alcoolate en C₁-C₆ de métal alcalin, le carbonate de thallium(I), un alcoolate en C₁-C₆ de thallium(I), l'hydroxyde de thallium(I) et une tri(alkyle en C₁-C₄)amine, et leurs mélanges.

4. Procédé selon la revendication 3, dans lequel le catalyseur au palladium est le bis(dibenzylidèneacétone)-palladium(0) et la base est un carbonate de métal alcalin.

5. Procédé selon la revendication 1, dans lequel le composé de formule III est amené à réagir avec un acide boronique.

6. Procédé selon l'une quelconque des revendications précédentes, dans lequel la première étape de réaction est conduite dans un premier solvant choisi dans la classe formée par un hydrocarbure aromatique, un hydrocarbure aromatique halogéné, un amide d'acide carboxylique, un éther et un hydrocarbure halogéné, et leurs mélanges ; et la dernière étape de réaction est conduite dans un second solvant choisi dans la classe formée par un hydrocarbure aromatique, un hydrocarbure aromatique halogéné, un amide d'acide carboxylique, un glycol, un alcool en C₁- C₄, une cétone et un éther, et leurs mélanges et mélanges avec l'eau.

7. Procédé selon la revendication 6, dans lequel le premier solvant est un hydrocarbure halogéné et le second solvant est le toluène ou un mélange toluène/éthanol.

8. Procédé selon l'une quelconque des revendications précédentes, dans lequel le composé de formule II est amené à réagir avec l'agent de bromation à une température de 50°C à 130°C, et le composé de formule III est amené à réagir avec le catalyseur au palladium, la base et le composé de formule IV, V ou VI à une température de 50°C à 130°C.

9. Procédé pour la préparation d'un composé oléfinique fluoré ayant la formule structurale I 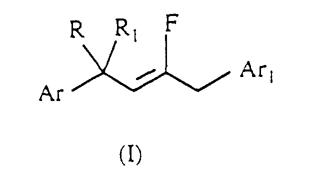 dans laquelle
R est l'hydrogène ou un groupe alkyle en C₁-C₄, et
R₁ est un groupe alkyle en C₁-C₄ ou cyclopropyle, ou bien R et R₁ sont pris avec l'atome de carbone auquel ils sont liés pour former un groupe cyclopropyle ;
Ar est un groupe phényle facultativement substitué par toute association d'un à trois groupes halogéno, alkyle en C₁-C₄, halogénoalkyle en C₁-C₄, alcoxy en C₁-C₄ ou halogénoalcoxy en C₁-C₄, ou
un groupe 1- ou 2-naphtyle facultativement substitué par toute association d'un à trois groupes halogéno, alkyle en C₁-C₄, halogénoalkyle en C₁-C₄, alcoxy en C₁-C₄ ou halogénoalcoxy en C₁-C₄ ;
Ar₁ est un groupe phénoxyphényle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄,
un groupe biphénylyle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄,
un groupe benzylphényle facultativement substitué par toute association d'un à cinq groupes halogéno, alkyle en C₁-C₄, halogénoalkyle en C₁-C₄, alcoxy en C₁-C₄ ou halogénoalcoxy en C₁-C₄, ou
un groupe benzoylphényle facultativement substitué par toute association d'un à cinq groupes halogéno, alkyle en C₁-C₄, halogénoalkyle en C₁-C₄, alcoxy
en C₁-C₄ ou halogénoalcoxy en C₁-C₄ ; et la configuration des groupes ArCRR₁- et -CH₂Ar₁ entre eux
autour de la double liaison est majoritairement *trans*, lequel procédé consiste à faire réagir un composé de 4-aryl-1-bromo-2-fluoro-2-butène ayant la formule structurale III 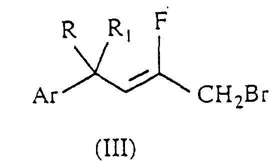 dans laquelle Ar, R et R₁ sont tels que définis ci-dessus, avec 0,001 à 0,1 équivalent molaire d'un catalyseur au palladium, au moins 2 équivalents molaires d'une base, et un acide boronique ayant la formule structurale IV, un anhydride boronique ayant la formule structurale V ou un ester borique ayant la formule structurale VI
(HO)₂B-Ar₁ (IV) 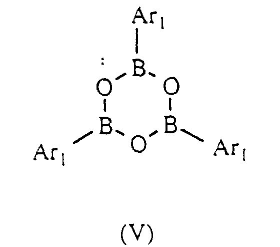
(R₂O)₂B-Ar₁ (VI)
où R₂ est un groupe alkyle en C₁-C₄ et Ar₁ est tel que défini ci-dessus, à une température de 50 à 130°C en présence d'un solvant.

10. Procédé selon la revendication 9, dans lequel le catalyseur au palladium est choisi dans la classe formée par le bis(dibenzylidèneacétone)palladium(0), le tétrakis-(triphénylphosphine)palladium(0), le chlorure de bis(acétonitrile)palladium(II), le chlorure de bis(triphénylphosphine)palladium(II), le dichlorure de [1,4 -bis(diphénylphosphine)butane]palladium(II), le diacétate de [1,1'-bis-(diphénylphosphino)ferrocène]palladium(II), l'acétate de palladium(II), le chlorure de palladium(II) et le palladium sur charbon activé, et leurs mélanges ; et la base est choisie dans la classe formée par un carbonate de métal alcalin, un carbonate de métal alcalino-terreux, un hydrogénocarbonate de métal alcalin, un hydroxyde de métal alcalin, un hydroxyde de métal alcalino-terreux, un alcoolate en C₁-C₆ de métal alcalin, le carbonate de thallium(I), un alcoolate en C₁-C₆ de thallium(I), l'hydroxyde de thallium(I) et une tri(alkyle en C₁-C₄)amine, et leurs mélanges.

11. Procédé selon la revendication 10, dans lequel le catalyseur au palladium est le bis (dibenzylidèneacétone)-palladium(0) et la base est un carbonate de métal alcalin.

12. Procédé selon l'une quelconque des revendications 9 à 11, dans lequel le solvant est choisi dans la classe formée par un hydrocarbure aromatique, un hydrocarbure aromatique halogéné, un amide d'acide carboxylique, un glycol, un alcool en C₁-C₄, une cétone et un éther, et leurs mélanges et mélanges avec l'eau.

13. Composé ayant la formule structurale III 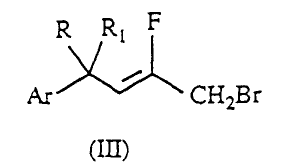 dans laquelle
R est l'hydrogène ou un groupe alkyle en C₁-C₄, ou bien R et R₁, pris avec l'atome de carbone auquel ils sont liés, forment un groupe cyclopropyle ;
R₁ est un groupe alkyle en C₁-C₄ ou cyclopropyle, ou bien R et R₁ sont pris avec l'atome de carbone auquel ils sont liés pour former un groupe cyclopropyle ;
Ar est un groupe phényle facultativement substitué par toute association d'un à trois groupes halogéno, alkyle en C₁-C₄, halogénoalkyle en C₁-C₄, alcoxy en C₁-C₄ ou halogénoalcoxy en C₁-C₄, ou
un groupe 1- ou 2-naphtyle facultativement substitué par toute association d'un à trois groupes halogéno, alkyle en C₁-C₄, halogénoalkyle en C₁-C₄, alcoxy en C₁-C₄ ou halogénoalcoxy en C₁-C₄ ; et
la configuration des groupes ArCRR₁- et -CH₂Br entre eux autour de la double liaison est majoritairement *trans.*

14. Composé selon la revendication 13, choisi dans la classe formée par :
le 1-(*p*-chlorophényl)-1-(3-bromo-2-fluoropropényl)cyclopropane, (Z) - ; et
le 1-bromo-4-(*p*-chlorophényl)-2-fluoro-4-méthyl-2-pentène, (Z)-.

15. Procédé pour la préparation d'un composé selon la revendication 13, lequel procédé consiste à faire réagir un composé de 4-aryl-2-fluoro-2-butène-1-ol ayant la formule structurale II 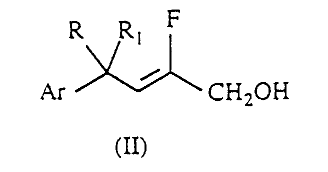 dans laquelle R, R₁ et Ar sont tels que définis dans la revendication 17, avec un agent de bromation choisi dans la classe formée par un complexe brome-triphénylphosphine, le tribromure de phosphore, le bromure de thionyle, HBr et leurs mélanges, dans un solvant à une température de 50 à 130°C.
